(19) Europäisches Patentamt
European Patent Office
Office européen des brevets

(11) EP 3 860 971 B1

(12) # FASCICULE DE BREVET EUROPEEN

(45) Date de publication et mention de la délivrance du brevet:
**10.08.2022 Bulletin 2022/32**

(21) Numéro de dépôt: **19790666.2**

(22) Date de dépôt: **18.09.2019**

(51) Classification Internationale des Brevets (IPC):
***C07C 213/10** (2006.01)* ***C07C 213/06** (2006.01)*
***C07C 219/08** (2006.01)* ***C07B 63/04** (2006.01)*

(52) Classification Coopérative des Brevets (CPC):
(C-Sets disponibles)
**C07C 213/06; C07B 63/04; C07C 213/10** (Cont.)

(86) Numéro de dépôt international:
**PCT/FR2019/052173**

(87) Numéro de publication internationale:
**WO 2020/070403 (09.04.2020 Gazette 2020/15)**

(54) **STABILISATION DE (METH)ACRYLATES D'AMINOALKYLE**
STABILISIERUNG VON AMINOALKYL(METH)ACRYLAT
AMINOALKYL (METH)ACRYLATE STABILISATION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **02.10.2018 FR 1859127**

(43) Date de publication de la demande:
**11.08.2021 Bulletin 2021/32**

(73) Titulaire: **ARKEMA FRANCE**
**92700 Colombes (FR)**

(72) Inventeurs:
- **BELLINI, Clément**
**57501 SAINT ALVOLD cedex (FR)**
- **CABON, Yves**
**57501 SAINT ALVOLD cedex (FR)**
- **DEFER, Patrice**
**57501 SAINT ALVOLD cedex (FR)**

(74) Mandataire: **Arkema Patent**
**Arkema France**
**DRD-DPI**
**420, rue d'Estienne d'Orves**
**92705 Colombes Cedex (FR)**

(56) Documents cités:
**WO-A1-2006/040470** **WO-A1-2012/076783**
**WO-A1-2018/104677**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



(Cont. page suivante)

(52) Classification Coopérative des Brevets (CPC):
(Cont.)

C-Sets
**C07C 213/06, C07C 219/08;**
**C07C 213/10, C07C 219/08**

## Description

### DOMAINE TECHNIQUE

La présente invention concerne la production de (méth)acrylates d'aminoalkyle par transestérification.

La transestérification implique l'utilisation d'une composition stabilisante comprenant au moins un composé N-oxyl et au moins un inhibiteur de polymérisation choisi parmi les inhibiteurs de polymérisation conventionnels dans un procédé de synthèse de (méth)acrylates d'aminoalkyle par transestérification pour inhiber en même temps la dégradation du catalyseur de transestérification ainsi que la polymérisation radicalaire du (méth)acrylate d'aminoalkyle.

### ART ANTERIEUR ET PROBLEME TECHNIQUE

Il est connu de de préparer les esters (méth)acryliques de formule (I) :

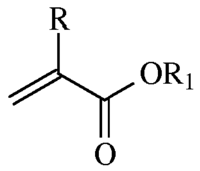

(I)

dans laquelle R est un atome d'hydrogène ou un groupement méthyle, et $R_1$ pouvant être un radical alkyle linéaire ou ramifié, ou un radical aliphatique cyclique, aryle, alkyle-aryle ou aryle-alkyle, pouvant contenir des hétéroatomes tel que l'atome d'azote, selon un procédé de transestérification par réaction d'un (méth)acrylate d'alkyle de formule (II) :

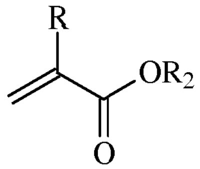

(II)

dans laquelle R a la signification précitée et $R_2$ pouvant être un groupement alkyle linéaire ou ramifié ayant de 1 à 4 atomes de carbone, avec un alcool de formule (III) :

$R_1$-OH (III)

dans laquelle $R_1$ a la signification précitée.

La réaction de transestérification s'effectue en présence d'un catalyseur de transestérification, généralement en présence d'un complexe organométallique.

La mise en œuvre d'un tel procédé se heurte cependant à des problèmes de polymérisation. Il est en effet bien connu que l'un des points délicats de la fabrication et/ou de la purification de monomères (méth)acryliques provient du fait que ces composés sont instables et ont tendance à évoluer facilement vers la formation de polymères. Cette évolution, causée par une réaction radicalaire due à l'effet de la température, est particulièrement favorisée lors des étapes de synthèse et de purification de ces monomères, par exemple dans les étapes de distillation. Il en résulte la formation, dans les équipements des installations, de dépôts de polymères solides qui finissent par provoquer des bouchages et rendent nécessaire un arrêt de l'atelier en vue de nettoyage.

Des inhibiteurs de polymérisation sont donc mis en œuvre pour stabiliser le milieu réactionnel dans les procédés de synthèse d'esters (méth)acryliques, en particulier dans les procédés de synthèse de (méth)acrylates de dialkylaminoalkyle par transestérification.

Les inhibiteurs de polymérisation conventionnels sont généralement choisis parmi les composés N-oxyls, comme le 2,2,6,6-tétraméthylpipéridine-N-oxyl et ses dérivés, les phénols ou naphtols comme le 2,6-tert-butyl-p-crésol, l'hydroquinone ou le monométhyl éther d'hydroquinone, les amines aromatiques comme la N,N-diphénylamine, les composés phosphorés comme le triphénylphosphite, et les composés soufrés comme la phénothiazine.

Ces composés peuvent être utilisés seuls ou en mélange, en particulier l'utilisation de la phénothiazine en excès avec du 4-hydroxy-2,2,6,6-tétraméthylpipéridine-N-oxyl (dénommé 4-HT) est décrite dans les documents US

2004/0171868 et US 2004/0168903.

Dans le document EP 467 850, il est préconisé d'utiliser un mélange de phénothiazine avec une amine telle que la N,N-diéthylhydroxylamine ou la N,N-di-tert-butylhydroxyamine pour éviter le colmatage d'équipements utilisés dans des procédés de synthèse de monomères à insaturations éthyléniques.

Les dérivés N-oxyls tels que le 2,2,6,6-tétraméthylpipéridine-N-oxyl et ses dérivés, sont des inhibiteurs de polymérisation reconnus pour leur efficacité vis-à-vis des monomères (méth)acryliques, qui peut être supérieure à l'efficacité stabilisante des inhibiteurs de polymérisation classiques de type hydroquinone ou phénothiazine.

Le document DE19956509A1 illustre les propriétés stabilisantes du 4-hydroxy 2,2,6,6-tétraméthylpipéridine-N-oxyl (4-HT) pour inhiber la polymérisation de l'acide acrylique. Ce dérivé peut être utilisé seul, ou, lorsque les températures du procédé sont élevées, en association avec de la phénothiazine afin de maintenir une bonne stabilité de la composition d'inhibiteur.

Selon le document EP 765 856, des compositions stabilisées d'acide acrylique sont obtenues à l'aide d'un mélange de 4-hydroxy 2,2,6,6-tétraméthylpipéridinyloxy (4-HT) et d'un dérivé phénol tel que l'éther méthylique d'hydroquinone.

Le document US 5,322,960 décrit la stabilisation de l'acide (méth)acrylique et de ses esters à l'aide de mélanges d'un composé N-oxyl, d'un composé phénolique et d'un composé phénothiazine. Les exemples illustrent l'utilisation de 4-HT / hydroquinone / phénothiazine seuls ou en mélange par paires. Ce document enseigne que la stabilisation est améliorée en présence du mélange ternaire de stabilisants.

WO 2006/040470 décrit un procédé pour la préparation d'acrylate de diméthylaminoéthyle à partir d'acrylate d'éthyle et de diméthyl aminoéthanol avec du titanate d'éthyle en tant que catalyseur. Les inhibiteurs de polymérisation peuvent être choisis parmi la phénothiazine (PTZ), le tétraméthyl-4-hydroxy-1-pipéridinyloxy (4-HO-TEMPO), le tertiobutylcatécol, le 2-orthoditertiobutylparacrésol (BHT), l'éther méthylique de l'hydroquinone (EMHQ), l'hydroquinone et/ou leurs mélanges.

Lorsqu'un composé N-oxyl est utilisé comme seul inhibiteur de polymérisation au cours de la synthèse de (méth)acrylates d'aminoalkyle par réaction de transestérification, la Société Déposante a constaté l'apparition de composés solides insolubles malgré l'efficacité stabilisante d'un tel composé vis-à-vis de la polymérisation. Ces solides ont été identifiés comme des sels inorganiques issus de la dégradation du catalyseur de transestérification. La formation de ces solides conduit à un encrassement du réacteur de transestérification qui se traduit par des travaux de maintenance sur une unité de production industrielle. De plus, il a été constaté que l'activité du complexe organométallique mis en œuvre comme catalyseur de transestérification est abaissée dans ces conditions, avec pour conséquence une baisse du rendement de la réaction.

De façon surprenante, les inventeurs ont trouvé que ce problème peut être résolu en associant au composé N-oxyl au moins un inhibiteur de polymérisation choisi parmi les composés phénoliques et les composés phénothiazine dans un rapport massique compris entre 1 et 10, bornes comprises. Les inventeurs on en effet découvert que ceci permet à la fois d'inhiber les réactions de polymérisation radicalaire au cours de la synthèse de (méth)acrylates d'aminoalkyle par transestérification, mais aussi d'éviter la dégradation du catalyseur de transestérification de type complexe organométallique et ainsi de maintenir une activité catalytique élevée au cours de la synthèse, tout en réduisant les travaux de maintenance de l'atelier de fabrication.

Même s'il est connu de l'art antérieur d'associer un composé N-oxyl avec un composé phénolique comme l'hydroquinone et/ou la phénothiazine des documents EP 0765 856 ; EP 0620 206 ; DE19956509 A1, il s'agit uniquement de la stabilisation des acides (méth)acryliques et de leurs esters vis-à-vis de la polymérisation radicalaire conduisant à la formation de sous-produits lourds. Le problème lié à la dégradation du catalyseur au cours de la synthèse par transestérification n'est pas posé ni même suggéré.

La présente invention fournit ainsi une composition d'inhibiteurs de polymérisation efficace pour empêcher la formation de sous-produits lourds issus de réactions de polymérisation radicalaire tout en évitant la dégradation du catalyseur au cours de la synthèse de (méth)acrylates d'aminoalkyle par transesterification.

RESUME DE L'INVENTION

L'invention a donc pour objet un procédé de synthèse de (méth)acrylates d'aminoalkyle, par transestérification d'un (méth)acrylate d'alkyle de formule (II) :

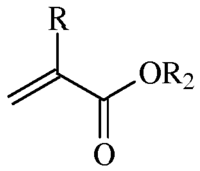

(II)

dans laquelle R est un atome d'hydrogène ou un groupement méthyle et $R_2$ pouvant être un groupement alkyle linéaire ou ramifié ayant de 1 à 4 atomes de carbone, avec un alcool $R_1$-OH de formule (III) :

HO-A - N $(R'_1)(R'_2)$ (III)

dans laquelle :

- A est un radical alkylène, linéaire ou ramifié, en $C_1$-$C_5$
- $R'_1$ et $R'_2$, identiques ou différents l'un de l'autre, représentent un atome d'hydrogène ou un radical alkyle en $C_1$-$C_4$. De préférence, $R'_1$ et $R'_2$ représentent un radical alkyle en $C_1$-$C_4$

en présence d'un catalyseur de transestérification choisi parmi les composés organométalliques répondant à l'une des formules suivantes:

- $M(OR_3)_4$ dans lequel $R_3$ est un radical alkyle, linéaire ou ramifié, en $C_1$-$C_5$ et M est un métal du groupe IV tels que Ti, Zr, Hf
- $M\{OC(CH_3)=CH-C(CH_3)=O\}_4$ dans laquelle M est un métal du groupe IV tels que Ti, Zr, Hf caractérisé en ce que la transestérification est effectuée en présence d'une composition stabilisante comprenant au moins un dérivé N-oxyl et au moins un inhibiteur de polymérisation choisi parmi les composés phénoliques et les composés phénothiazine dans un rapport massique compris entre 1 et 10, bornes comprises.

Selon un mode de réalisation de l'invention, le composé N-oxyl est choisi parmi le 2,2,6,6-tétraméthylpipéridine 1-oxyl (dénommé TEMPO) , et ses dérivés tels que le 4-Hydroxy-2,2,6,6-tétraméthylpipéridine 1-oxyl (4-OH-TEMPO ou 4-HT), ou le 4-oxo-2,2,6,6-tétraméthylpipéridine 1-oxyl (4-Oxo-TEMPO), ainsi qu'un mélange de ces composés.

De préférence, le composé N-oxyl est le 4-Hydroxy-2,2,6,6-tétraméthylpipéridine 1-oxyl (4-OH-TEMPO ou 4-HT) (Numéro CAS 2226-96-2) dont la structure est la suivante :

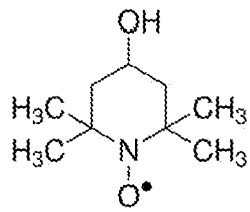

Par composé phénolique on entend les composés dérivés du phénol, du naphtol et les quinones.

Comme composés phénoliques, on peut citer les composés suivants sans que cette liste soit limitative : p-aminophénol, p-nitrosophénol, 2-tert-butylphénol, 4-tert-butylphénol, 2,4-di-tert-butylphénol, 2-méthyl-4-tert-butyéphénol, 4-méthyl-2,6-tert-butylphénol (ou 2,6-tert-butyl-p-crésol) or 4-tert-butyl-2,6-diméthylphénol, hydroquinone (HQ), éther méthylique d'hydroquinone (EMHQ).

Par composé phénothiazine, on entend la phénothiazine et ses dérivés, de préférence la phénothiazine (PTZ).

De préférence, la composition stabilisante comprend au moins un inhibiteur de polymérisation choisi parmi la phénothiazine, le 4-méthyl-2,6-tert-butylphénol, l'hydroquinone et l'éther méthylique d'hydroquinone.

Selon un mode de réalisation, le rapport massique entre le composé N-oxyl et l'(ou les) inhibiteur(s) de polymérisation est compris de préférence entre 2 et 10, plus préférentiellement entre 4 et 10, en particulier entre 5 et 10, le rapport massique étant exprimé bornes comprises.

Selon un mode de réalisation, le catalyseur de transestérification est un complexe organométallique à base de titane.

Selon un mode de réalisation, le catalyseur de transestérification répond à l'une des formules suivantes :

- $M(OR_3)_4$ dans laquelle $R_3$ est un radical alkyle, linéaire ou ramifié, en $C_1$-$C_5$ et M est un métal du groupe IV tels que Ti, Zr, Hf.
- $M\{OC(CH_3)=CH-C(CH_3)=O\}_4$ dans laquelle M est métal du groupe IV tels que Ti, Zr, Hf.

Comme catalyseurs de transestérification, on peut citer en particulier les alcoolates de titane, comme les titanates de tétraalkyle (éthyle, n-propyle, isopropyle, n-butyle), ou les acétylacétonates de Ti, Zr, Hf.
Selon l'invention, l'association d'un inhibiteur de polymérisation avec le composé N-oxyl permet d'inhiber la dégradation du catalyseur organométallique observée lorsque le composé N-oxyl est utilisé seul comme inhibiteur de polymérisation dans un procédé de synthèse de (méth)acrylates d'aminoalkyle par transestérification, de préférence dans un procédé de synthèse de (méth)acrylates de dialkyl aminoalkyle par transestérification. De plus, il a été constaté que cette association est beaucoup plus efficace pour inhiber la polymérisation que l'inhibiteur de polymérisation seul.
Dans la présente invention, « composition stabilisante » signifie que les réactions secondaires de polymérisation radicalaire ainsi que la décomposition du catalyseur métallique sont inhibées dans le mélange réactionnel en présence de ladite composition stabilisante.
L'invention fournit ainsi un procédé de synthèse de (méth)acrylates d'aminoalkyle pouvant fonctionner en continu sur une longue durée sans perte d'efficacité du catalyseur et sans nécessiter de nettoyage du réacteur
L'invention est maintenant décrite plus en détail et de façon non limitative dans la description qui suit.

EXPOSE DETAILLE DE L'INVENTION

L'invention a trait à la synthèse de (méth)acrylates d'aminoalkyle de formule (I) :

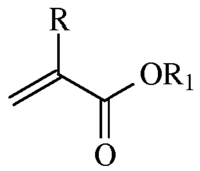

(I)

par réaction de transestérification d'un (méth)acrylate d'alkyle de formule (II) :

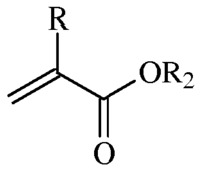

(II)

avec un alcool de formule : $R_1$-OH, telle que définie à la revendication 1.
De préférence, l'alcool $R_1$-OH est le N,N-diméthyl aminoéthanol (DMAE), dénommé aussi dans la suite de l'exposé par diméthyl aminoéthanol.
De préférence, le (méth)acrylate d'alkyle de formule (II) est l'acrylate de méthyle ou l'acrylate d'éthyle, plus préférentiellement l'acrylate d'éthyle.
Selon un mode de réalisation, l'invention porte sur la synthèse d'acrylate de diméthyl aminoéthyle (ADAME) par réaction de transestérification d'acrylate d'éthyle (AE) avec du diméthylaminol éthanol.
Le catalyseur de transestérification est un complexe organométallique, utilisé généralement à une teneur pouvant aller de 0,001 à 0,02 mole, de préférence de 0,005 à 0,01 mole par mole d'alcool $R_1$-OH.
De préférence, le catalyseur de transestérification est un complexe organométallique à base de titane.
La température de réaction est généralement comprise entre 80 et 150°C et la pression est généralement maintenue entre 0,5 et 1,1 bar. De préférence, la température de réaction est comprise entre 90 et 130°C et la pression est de l'ordre de 0,65 bar à la pression atmosphérique
Selon l'invention, la composition stabilisante comprenant au moins un dérivé N-oxyl et au moins un inhibiteur de polymérisation autre qu'un dérivé N-oxyl permet d'empêcher les réactions secondaires de polymérisation radicalaire au cours de la synthèse tout en évitant la décomposition du catalyseur en sels insolubles.
La composition stabilisante est généralement introduite dans le réacteur de transestérification sous forme d'une solution dans le (méth)acrylate d'alkyle (II), à raison de 100 à 5000 ppm, de préférence entre 500 et 3000 ppm, par

rapport à la charge initiale de réactifs.

Le composé N-oxyl est de préférence en excès dans la composition stabilisante, généralement, la teneur massique en composé N-oxyl est supérieure à 50%, de préférence supérieure à 80%, et peut aller de 80% à 95% par rapport à la composition stabilisante.

L'invention procure l'avantage de mettre en œuvre une composition stabilisante plus performante qu'un inhibiteur de polymérisation seul vis-à-vis de la stabilisation des monomères (méthacryliques, sans impact négatif sur le procédé par transestérification.

Les exemples suivants illustrent la présente invention sans toutefois en limiter la portée.

## PARTIE EXPERIMENTALE

Dans les exemples suivants, toutes les concentrations sont données en % massique et ppm massique sauf mention contraire, et les abréviations suivantes ont été utilisées :

AE : Acrylate d'éthyle
DMAE : Diméthyl aminoéthanol
ADAME : Acrylate de diméthyl aminoéthyle
PTZ : Phénothiazine
4-HT : 4-OH-TEMPO
EMHQ : Ether méthylique d'hydroquinone
$Ti(OEt)_4$ : titanate de tétraéthyle
TOF : Turn Over Frequency
MES : Matières en suspension

Le protocole suivant a été utilisé pour l'ensemble des exemples selon l'invention et les exemples comparatifs.

Dans un réacteur agité de 0,5 L, chauffé par circulation d'huile thermostatée à 135°C dans une double enveloppe, surmonté d'une colonne à distiller à garnissage Multiknit, avec condenseur à eau glycolée en tête de colonne, tête de reflux, séparateur à vide, recettes et pièges, on introduit en continu l'AE, l'alcool lourd (DMAE) et un catalyseur de transestérification ($Ti(OEt)_4$).

Les inhibiteurs de polymérisation (PTZ, 4-HT, EMHQ ou un mélange de ces composés) sont injectés en continu en mélange avec l'AE.

Durant toute la synthèse, on effectue un bullage d'air dans le mélange réactionnel. La réaction est effectuée à une température de 119-121 °C sous un vide de 114657.264-115990.488 Pa (860-870 mm Hg).

L'éthanol formé au cours de la réaction est éliminé au fur et à mesure de sa formation sous forme d'un azéotrope AE/éthanol.

Le taux de conversion est suivi par analyse réfractométrique de l'azéotrope. La teneur en éthanol est comprise entre 58% et 62%.

Le mélange brut réactionnel est extrait à l'aide d'un débordement et récupéré dans une recette pour être analysé après 120 h de marche.

Les flux de distillat et de pied ont été analysés par Chromatographie en phase gaz, afin de déterminer le rendement en ester désiré et la conversion de l'alcool lourd.

Les inhibiteurs ont été analysés et quantifiés par chromatographie en phase liquide.

La quantification des impuretés lourdes (catalyseur + sous-produits lourds formés) a été réalisée à l'aide d'une balance thermique.

A partir de ces analyses, l'activité catalytique au bout de 120 h est également déterminée par la valeur du TOF, calculée selon la formule ci-dessous. Le temps de séjour TdS est déterminé en fonction du débit d'extraction en pied du réacteur de transestérification.

$$\boldsymbol{TOF} = \frac{\boldsymbol{N_{ADAME}}\ (moles)}{\boldsymbol{N_{Catalyseur}}\ (\text{moles}) \times \boldsymbol{TdS}}$$

Des essais selon l'invention ont été réalisés en utilisant comme inhibiteur de polymérisation soit un mélange de 4-HT et de PTZ en excès de 4-HT (exemples 1 à 3) soit un mélange de 4-HT et de EMHQ en excès de 4-HT (exemple 4).

A titre de comparaison, des essais ont été réalisés avec du 4-HT seul (exemple 5), de la PTZ seule (exemple 6), ou un mélange de 4-HT et de PTZ en excès de PTZ (exemple 7). Pour l'ensemble des essais selon l'invention (Exemples 1 à 4), le réacteur est resté totalement limpide après 120 h de marche et aucun encrassement du réacteur n'a été observé.

Les essais comparatifs réalisés avec le 4-HT seul (Ex 5) ont mis en évidence l'apparition de composé inorganique blanc s'accumulant au cours de la synthèse, avec encrassement du réacteur dès 24h de marche et perte d'activité du catalyseur.

Les essais comparatifs réalisés avec la PTZ seule (Ex 6) ou un mélange de 4-HT et de PTZ en excès de PTZ (Ex. 7) ont mis en évidence la formation plus importante de polymères solubles (lourds > 1,0%) dans le réacteur de transestérification.

Les résultats de ces essais sont rassemblés dans le tableau 1 ci-après.

| | | Ex 1 | Ex 2 | Ex 3 | Ex 4 | Ex 5 comp | Ex 6 comp | Ex 7 comp |
|---|---|---|---|---|---|---|---|---|
| ALIMENTATION | AE (g/h) | 61,8 | 62,2 | 62,2 | 61,5 | 62,0 | 59,9 | 59,9 |
| | DMAE (g/h) | 34,3 | 34,5 | 34,5 | 34,2 | 34,4 | 33,3 | 33,3 |
| | $Ti(OEt)_4$ (g/h) | 1,2 | 1,2 | 1,2 | 1,2 | 1,2 | 1,2 | 1,2 |
| STAB | PTZ (g/h) | 0,004 | 0,008 | 0,020 | - | - | 0,05 | 0,05 |
| | EMHQ (g/h) | - | - | - | 0,008 | - | - | - |
| | 4-HT (g/h) | 0,039 | 0,041 | 0,040 | 0,040 | 0,040 | - | 0,012 |
| RESULTATS | Conversion (%) | 83,0 | 83,7 | 83,2 | 79,9 | 83,5 | 83,1 | 83,0 |
| | Rendement (%) | 84,1 | 84,2 | 84,3 | 83,0 | 81,6 | 82,8 | 82,9 |
| | TOF ($h^{-1}$) | 18,1 | 17,8 | 17,8 | 19,1 | 15,9 | 16,7 | 17,1 |
| | Lourds (%) | 0,7 | 0,8 | 0,6 | 0,9 | 1,0 | 1,4 | 1,6 |
| | Encrassement (h) | > 120 | > 120 | > 120 | > 120 | 24 | > 120 | > 120 |
| | Aspect du réacteur | limpide | limpide | limpide | limpide | MES | limpide | limpide |

Tableau 1

## Revendications

1. Procédé de synthèse de (méth)acrylates d' aminoalkyle, par transestérification d'un (méth)acrylate d'alkyle de formule (II) :

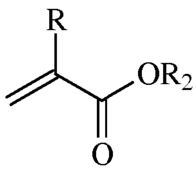

(II)

dans laquelle R est un atome d'hydrogène ou un groupement méthyle et $R_2$ pouvant être un groupement alkyle linéaire ou ramifié ayant de 1 à 4 atomes de carbone,
avec un alcool $R_1$-OH de formule (III) :

(III) HO-A - N (R'$_1$)(R'$_2$)

dans laquelle

- A est un radical alkylène, linéaire ou ramifié, en $C_1$-$C_5$
- R'$_1$ et R'$_2$, identiques ou différents l'un de l'autre, représentent un atome d'hydrogène ou un radical alkyle en $C_1$-$C_4$, de préférence un radical alkyle en $C_1$-$C_4$ en présence d'un catalyseur de transestérification choisi parmi les composés organométalliques répondant à l'une des formules suivantes :

  • $M(OR_3)_4$ dans lequel $R_3$ est un radical alkyle, linéaire ou ramifié, en $C_1$-$C_5$ et M est un métal du groupe IV tels que Ti, Zr, Hf
  • $M\{OC(CH_3)=CH-C(CH_3)=O\}_4$ dans laquelle M est un métal du groupe IV tels que Ti, Zr, Hf.

**caractérisé en ce que** la transestérification est effectuée en présence d'une composition stabilisante comprenant au moins un dérivé N-oxyl et au moins un inhibiteur de polymérisation choisi parmi les composés phénoliques et les composés phénothiazine dans un rapport massique compris entre 1 et 10, bornes comprises.

2. Procédé selon la revendication 1 **caractérisé en ce que** le (méth)acrylate d'aminoalkyle est l'acrylate de diméthyl aminoéthyle.

3. Procédé selon l'une des revendications 1 ou 2 dans lequel le composé N-oxyl est choisi parmi le 2,2,6,6-tétraméthylpipéridine 1-oxyl (dénommé TEMPO), et ses dérivés tels que le 4-Hydroxy-2,2,6,6-tétraméthylpipéridine 1-oxyl (4-OH-TEMPO) ou le 4-oxo-2,2,6,6-tétraméthylpipéridine 1-oxyl (4-Oxo-TEMPO), ainsi qu'un mélange de ces composés.

4. Procédé selon l'une quelconque des revendications précédentes dans lequel la composition stabilisante comprend au moins un inhibiteur de polymérisation choisi parmi la phénothiazine, le 4-méthyl-2,6-tert-butylphénol, l'hydroquinone et l'éther méthylique d'hydroquinone.

5. Procédé selon l'une quelconque des revendications précédentes dans lequel le rapport massique entre le composé N-oxyl et l'(ou les) inhibiteur(s) de polymérisation est compris entre 2 et 10, bornes comprises.

6. Procédé selon l'une quelconque des revendications précédentes dans lequel le rapport massique entre le composé N-oxyl et l'(ou les) inhibiteur(s) de polymérisation est compris entre 4 et 10, bornes comprises.

7. Procédé selon l'une quelconque des revendications précédentes dans lequel le rapport massique entre le composé N-oxyl et l'(ou les) inhibiteur(s) de polymérisation est compris entre 5 et 10, bornes comprises.

8. Procédé selon l'une quelconque des revendications précédentes dans lequel l'alcool $R_1$-OH est le diméthyl aminoéthanol.

9. Procédé selon l'une quelconque des revendications précédentes dans lequel ledit métal M du groupe IV est le titane.

10. Procédé selon l'une quelconque des revendications 1 à 8 dans lequel ledit catalyseur de transestérification est choisi parmi les alcoolates de titane, comme les titanates de tétraalkyle (éthyle, n-propyle, isopropyle, n-butyle), ou les acétylacétonates de Ti, Zr ou Hf.

## Patentansprüche

1. Verfahren zur Synthese von Aminoalkyl(meth)-acrylaten durch Umesterung eines Alkyl(meth)acrylats der Formel (II):

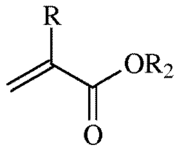

(II)

in der R für ein Wasserstoffatom oder eine Methylgruppe steht und $R_2$ für eine lineare oder verzweigte Alkylgruppe mit 1 bis 4 Kohlenstoffatomen stehen kann,
mit einem Alkohol $R_1$-OH der Formel (III):

(III) HO-A-N($R'_1$)($R'_2$)

in der

- A für einen linearen oder verzweigten $C_1$-$C_5$-Alkylenrest steht,
- $R'_1$ und $R'_2$, die gleich oder voneinander verschieden sind, für ein Wasserstoffatom oder einen $C_1$-$C_4$-Alkylrest, vorzugsweise einen $C_1$-$C_4$-Alkylrest, stehen, in Gegenwart eines Umesterungskatalysators, der aus einer der folgenden Formeln entsprechenden metallorganischen Verbindungen ausgewählt wird:

  • $M(OR_3)_4$, wobei $R_3$ für einen linearen oder verzweigten $C_1$-$C_5$-Alkylrest steht, M für ein Metall der Gruppe IV wie Ti, Zr, Hf steht,
  • $M\{OC(CH_3)=CH-C(CH_3)=O\}_4$, wobei M für ein Metall der Gruppe IV wie Ti, Zr, Hf steht,

**dadurch gekennzeichnet, dass** die Umesterung in Gegenwart einer Stabilisierungszusammensetzung, die mindestens ein N-Oxylderivat und mindestens einen aus Phenolverbindungen und Phenothiazinverbindungen ausgewählten Polymerisationsinhibitor in einem Massenverhältnis zwischen 1 und 10 einschließlich der Grenzen umfasst, durchgeführt wird.

2. Verfahren nach Anspruch 1, **dadurch gekennzeichnet, dass** es sich bei dem Aminoalkyl (meth) acrylat um Dimethylaminoethylacrylat handelt.

3. Verfahren nach Anspruch 1 oder 2, wobei die N-Oxylverbindung aus 2,2,6,6-Tetramethylpiperidin-1-oxyl (Bezeichnung TEMPO), dessen Derivaten wie 4-Hydroxy-2,2,6,6-tetramethylpiperidin-1-oxyl (4-OH-TEMPO) oder 4-Oxo-2,2,6,6-tetramethylpiperidin-1-oxyl (4-Oxo-TEMPO) sowie Mischungen dieser Verbindungen ausgewählt wird.

4. Verfahren nach einem der vorhergehenden Ansprüche, wobei die Stabilisierungszusammensetzung mindestens einen aus Phenothiazin, 4-Methyl-2,6-tert-butylphenol, Hydrochinon und Hydrochinonmethylether ausgewählten Polymerisationsinhibitor umfasst.

5. Verfahren nach einem der vorhergehenden Ansprüche, wobei das Massenverhältnis zwischen der N-Oxylverbindung und dem Polymerisationsinhibitor bzw. den Polymerisationsinhibitoren zwischen 2 und 10 einschließlich der Grenzen liegt.

6. Verfahren nach einem der vorhergehenden Ansprüche, wobei das Massenverhältnis zwischen der N-Oxylverbindung und dem Polymerisationsinhibitor bzw. den Polymerisationsinhibitoren zwischen 4 und 10 einschließlich der Grenzen liegt.

7. Verfahren nach einem der vorhergehenden Ansprüche, wobei das Massenverhältnis zwischen der N-Oxylverbindung und dem Polymerisationsinhibitor bzw. den Polymerisationsinhibitoren zwischen 5 und 10 einschließlich der Grenzen liegt.

8. Verfahren nach einem der vorhergehenden Ansprüche, wobei es sich bei dem Alkohol $R_1$-OH um Dimethylaminoethanol handelt.

9. Verfahren nach einem der vorhergehenden Ansprüche, wobei es sich bei dem Metall M der Gruppe IV um Titan handelt.

**10.** Verfahren nach einem der Ansprüche 1 bis 8, wobei der Umesterungskatalysator aus Titanalkoholaten, wie Tetraalkyltitanaten (Tetraethyl-, -n-propyl-, -isopropyl-, -n-butyltitanat), oder Ti-, Zr- oder Hf-Acetylacetonaten ausgewählt wird.

## Claims

**1.** A process for synthesizing aminoalkyl (meth)acrylates, by transesterification of an alkyl (meth)acrylate of formula (II):

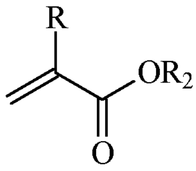

(II)

in which R is a hydrogen atom or a methyl group and $R_2$ can be a linear or branched alkyl group having from 1 to 4 carbon atoms,
with an alcohol $R_1$-OH of formula (III):

(III) HO-A - N $(R'_1)(R_2)$

in which

- A is a linear or branched $C_1$-$C_5$ alkylene radical,
- $R'_1$ and $R'_2$, which are identical or different from one another, represent a hydrogen atom or a $C_1$-$C_4$ alkyl radical, preferably a $C_1$-$C_4$ alkyl radical,

in the presence of a transesterification catalyst selected from organometallic compounds conforming to one of the following formulas:

• $M(OR_3)_4$ in which $R_3$ is a linear or branched $C_1$-$C_5$ alkyl radical and M is a group IV metal such as Ti, Zr, Hf,
• $M\{OC(CH_3)=CH-C(CH_3)=O\}_4$ in which M is a group IV metal such as Ti, Zr, Hf,

**characterized in that** the transesterification is carried out in the presence of a stabilizing composition comprising at least one N-oxyl derivative and at least one polymerization inhibitor selected from phenol compounds and phenothiazine compounds in a mass ratio of between 1 and 10, endpoints included.

**2.** The process as claimed in claim 1, **characterized in that** the aminoalkyl (meth)acrylate is dimethylaminoethyl acrylate.

**3.** The process as claimed in either of claims 1 and 2, in which the N-oxyl compound is selected from 2,2,6,6-tetramethylpiperidine 1-oxyl (called TEMPO), and derivatives thereof such as 4-hydroxy-2,2,6,6-tetramethylpiperidine 1-oxyl (4-OH-TEMPO) or 4-oxo-2,2,6,6-tetramethylpiperidine 1-oxyl (4-Oxo-TEMPO), and also a mixture of these compounds.

**4.** The process as claimed in any one of the preceding claims, in which the stabilizing composition comprises at least one polymerization inhibitor selected from phenothiazine, 4-methyl-2,6-tert-butylphenol, hydroquinone, and hydroquinone methyl ether.

**5.** The process as claimed in any one of the preceding claims, in which the mass ratio of the N-oxyl compound to the one or more polymerization inhibitors is between 2 and 10, endpoints included.

**6.** The process as claimed in any one of the preceding claims, in which the mass ratio of the N-oxyl compound to the one or more polymerization inhibitors is between 4 and 10, endpoints included.

**7.** The process as claimed in any one of the preceding claims, in which the mass ratio of the N-oxyl compound to the one or more polymerization inhibitors is between 5 and 10, endpoints included.

**8.** The process as claimed in any one of the preceding claims, in which the alcohol $R_1$-OH is dimethylaminoethanol.

**9.** The process as claimed in any one of the preceding claims, in which said group IV metal M is titanium.

**10.** The process as claimed in any one of claims 1 to 8, in which said transesterification catalyst is selected from titanium alkoxides, such as tetraalkyl (ethyl, n-propyl, isopropyl, n-butyl) titanates, or Ti, Zr or Hf acetylacetonates.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 20040171868 A
- US 20040168903 A
- EP 467850 A
- DE 19956509 A1
- EP 765856 A
- US 5322960 A
- WO 2006040470 A
- EP 0765856 A
- EP 0620206 A